(19) Europäisches Patentamt
European Patent Office
Office européen des brevets

(11) Veröffentlichungsnummer : **0 041 921**
**B1**

(12) # EUROPÄISCHE PATENTSCHRIFT

(45) Veröffentlichungstag der Patentschrift : **26.10.83**

(51) Int. Cl.³ : **C 07 C101/18**, C 07 C 99/00

(21) Anmeldenummer : **81810214.7**

(22) Anmeldetag : **02.06.81**

(54) **Verfahren zur Herstellung von N-(1'-Alkoxycarbonyläthyl)-2,6-dialkylanilinen.**

(30) Priorität : **09.06.80 US 157760**

(43) Veröffentlichungstag der Anmeldung : **16.12.81 Patentblatt 81/50**

(45) Bekanntmachung des Hinweises auf die Patenterteilung : **26.10.83 Patentblatt 83/43**

(84) Benannte Vertragsstaaten : **AT BE CH DE FR GB IT LI NL SE**

(56) Entgegenhaltungen :
**EP A 0 008 057**
**DE A 2 153 356**
**US A 4 025 648**

**SYNTHESIS, Juni 1976 STUTTGART (DE) V. BOCCHI u. a. : « Synthesis of N-alkylindoles using tetraalkylammonium salts catalysis », Seiten 414-416**

(73) Patentinhaber : **CIBA-GEIGY AG**
**Patentabteilung Postfach**
**CH-4002 Basel (CH)**

(72) Erfinder : **Grieder, Alfred, Dr.**
**Ob den Reben 15**
**CH-4461 Böckten (CH)**
Erfinder : **Coers, Klaus-Jürgen**
**Lochackerstrasse 10**
**CH-4153 Reinach (CH)**

**EP 0 041 921 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Verfahren zur Herstellung von N-(1'-Alkoxycarbonyläthyl)-2,6-dialkylanilinen

Die vorliegende Erfindung bezieht sich auf ein Verfahren zur Herstellung von N-(1'-Alkoxycarbonyläthyl)-2,6-dialkylanilinen der Formel I

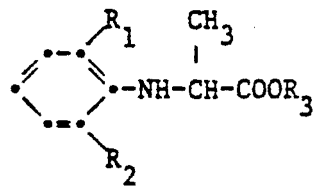

(I)

in welcher $R_1$ und $R_2$ je Methyl oder Aethyl bedeuten und $R_3$ für eine Alkylgruppe mit 1-4 Kohlenstoffatomen steht, durch Umsetzung eines 2,6-Dialkylanilins der Formel II

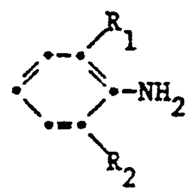

(II)

in welcher $R_1$ und $R_2$ die oben angegebene Bedeutung haben, mit einem 2-Brompropionsäurealkylester der Formel III

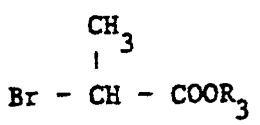

(III)

in welcher $R_3$ die oben angegebene Bedeutung hat.

Die N-(1'-Alkoxycarbonyläthyl)-2,6-dialkylaniline der Formel I sind wertvolle Zwischenprodukte für die Herstellung von pestizid wirksamen Verbindungen. Sie können beispielsweise durch Umsetzung mit Säurechloriden, wie Chloracetylchlorid, Methoxyacetylchlorid oder Furan-2-carbonsäurechlorid in entsprechende N-Acylaniline übergeführt werden, die sich durch eine hervorragende Wirkung gegen phytopathogene Mikroorganismen auszeichnen und daher ausgedehnte Anwendung im Pflanzenschutz finden. Solche N-Acylaniline, sowie ihre Herstellung und Verwendung sind beispielsweise in den US-Patenten 4,008,066, 4,094,990 und 4,151,299 beschrieben.

Unter den N-Acylanilinen der vorgenannten Art, die durch Acylierung der erfindungsgemäss herstellbaren N-(1'-Alkoxycarbonyläthyl)-2,6-dimethylaniline der Formel I hergestellt werden können, sind insbesondere das N-Methoxyacetyl-N-(1'-methoxycarbonyläthyl)-2,6-dimethylanilin und das N-(2"-Furoyl)-N-(1'-methoxycarbonyläthyl)-2,6-dimethylanilin zu nennen.

Die Herstellung von N-(1'-Alkoxycarbonyläthyl)-2,6-dialkylanilinen der Formel I durch Umsetzung von 2,6-Dialkylanilinen der Formel II mit α-Halogenpropionsäureestern der Formel III ist einerseits wegen der durch die orthoständigen Alkylgruppen bedingten sterischen Hinderung und andererseits wegen der Hydrolyseempfindlichkeit der α-Halogenpropionsäureester problematisch. Zur Lösung der mit der Durchführung dieses Verfahrens verbundenen Probleme wurden bereits verschiedene Vorschläge gemacht.

Es ist bekannt, N-Alkoxycarbonylmethyl-2,6-dialkylaniline durch Umsetzung von α-Halogenessigsäureestern und 2,6-Dialkylanilinen in Gegenwart von wässerigen Alkalimetallhydroxiden herzustellen. Nach dieser Methode werden die gewünschten Produkte in Folge von Nebenreaktionen, wie, N,N-Dialkylierung und Hydrolyse der Estergruppe, nur in ungenügender Reinheit und in unbefriedigenden Ausbeuten erhalten. (US Patent 3,882,162).

Es wurde bereits vorgeschlagen, die Nachteile des vorgenannten Verfahrens dadurch zu vermeiden, dass man die Umsetzung des 2,6-Dialkylanilins mit dem α-Halogencarbonsäureester in Gegenwart von überschüssigem 2,6-Dialkylanilin als säurebindendes Mittel und in Gegenwart einer katalytischen Menge des Hydrochlorids des betreffenden 2,6-Dialkylanilins bei Temperaturen von 100 bis 250 °C durchzuführen. Doch auch mit diesem Verfahren liegen die erreichbaren Ausbeuten unter 70 % der Theorie (US Patent 3,882,162).

Ferner wurde bereits vorgeschlagen, kernsubstituierte Aniline bei Temperaturen von 100 bis 175 °C in Gegenwart eines tertiären Amins als säurebindendes Mittel mit α-Halogencarbonsäureestern umzusetzen, wobei gemäss einer bevorzugten Ausführungsform dieses Verfahrens die Umsetzung in überschüssigem Ester als Lösungsmittel durchgeführt und zur Beschleunigung der Reaktion das tertiäre Amin bereits zu Beginn der Umsetzung in Form eines Salzes, beispielsweise in Form des Hydrochlorids, zugesetzt wird. Nach diesem Verfahren werden Umsätze an Anilin von 90 bis 96 %, eine Selektivität von 78 bis 90 % und Ausbeuten an N-(1'-Alkoxycarbonylalkyl)-anilinen von 70-86 % d. Th. erreicht, wobei die Ausbeuteangaben nicht auf tatsächlich isoliertem Reinprodukt, sondern auf analytischer Bestimmung des Gehalts an Reinprodukt im Rohprodukt beruhen. (CH Patent 572.017).

Weiterhin ist aus der US Patentschrift 4.008.066 bekannt, 2,6-Dimethylanilin bei 120 bis 125 °C in Gegenwart von Natriumhydrogencarbonat als säurebindende Mittel mit dem dreifach molaren Ueberschuss an 2-Brompropionsäuremethylester umzusetzen. Bei diesem Verfahren wird N-(1'-Methoxycarbonyläthyl)-2,6-dimethylanilin in einer Ausbeute von 79,6 % der Theorie erhalten.

Wie der vorstehende Ueberblick über den Stand der Technik zeigt, ist es mit den bisher bekannten Verfahren nicht möglich, N-(1'-Alkoxycarbonyläthyl)-2,6-dialkylaniline in befriedigender Ausbeute und Reinheit herzustellen. Es ist daher das Ziel der vorliegenden Erfindung, ein Verfahren vorzusehen, das die Nachteile der bekannten Verfahren vermeidet und das die Herstellung von N-(1'-Alkoxycarbonyläthyl)-2,6-dialkylanilinen der Formel I in zufriedenstellender Ausbeute und Reinheit auf einfache Weise ermöglicht.

Gemäss vorliegender Erfindung wird vorgeschlagen, dass man die Umsetzung des 2,6-Dialkylanilins der Formel II mit dem 2-Brompropionsäurealkylester der Formel III in überschüssigem 2,6-Dialkylanilin als Lösungsmittel bei 110 bis 125 °C in Gegenwart einer quaternären Verbindung der Formel IV

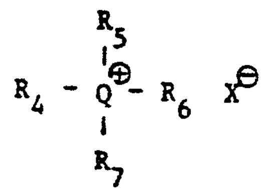

(IV)

in welcher Q Stickstoff oder Phosphor darstellt, die Reste $R_4$, $R_5$, $R_6$ und $R_7$ je einen Alkylrest mit 1 bis 16 Kohlenstoffatomen oder Phenyl bedeuten und einer der Reste $R_4$, $R_5$, $R_6$ und $R_7$ auch für Benzyl stehen kann, wobei, wenn Q Stickstoff bedeutet, Q zusammen mit dreien der Reste $R_4$, $R_5$, $R_6$ und $R_7$ auch einen Pyridinrest bedeuten kann, während der vierte Alkyl mit 1-16 Kohlenstoffatomen, Phenyl oder Benzyl darstellt, und $X^{\ominus}$ ein Halogenid- oder ein Bisulfatanion bedeutet und in Gegenwart eines Alkalimetallcarbonats oder -hydrogencarbonats als Säureakzeptor durchführt, während der Umsetzung das gebildete Wasser durch Destillation abtrennt und die nach Zugabe von Wasser und Phasentrennung erhaltene organische Phase destillativ aufarbeitet.

Das erfindungsgemäss im Ueberschuss zu verwendende 2,6-Dialkylanilin der Formel II wird vorteilhaft in Mengen von 1,5 bis 2,5 Mol pro Mol 2-Brompropionsäurealkylester der Formel III, eingesetzt. Die Verwendung eines geringeren oder grösseren Ueberschusses ist ebenfalls möglich. Die Verwendung eines geringeren Ueberschusses an 2,6-Dialkynanilin der Formel II als 1,5 Mol pro Mol 2-Brompropionsäurealkylester der Formel III ist jedoch im allgemeinen nicht zweckmässig, da bei einem derartig geringen Ueberschuss Ausbeute und Umsatz niedriger wird. Die Verwendung von mehr als 2,5 Mol 2,6-Dialkylanilin der Formel II pro Mol 2-Brompropionsäurealkylester der Formel III bringt in bezug auf Ausbeute und Qualität des N-(1'-Alkoxycarbonyläthyl)-2,6-dialkylanilins der Formel I keine Vorteile und ist wegen des mit der destillativen Entfernung des überschüssigen 2,6-Dialkylanilins verbundenen Aufwandes unwirtschaftlich. Vorzugsweise werden 1,6 bis 1,8 Mol 2,6-Dialkylanilin der Formel II pro Mol 2-Brompropionsäurealkylester der Formel III verwendet.

Als quaternäre Verbindung der Formel IV kommen die Chloride, Bromide und Jodide und die Bisulfate in Betracht. Die quaternären Verbindungen der Formel IV werden in der Regel in Mengen von 0,5 bis 5 Gew.%, bezogen auf den 2-Brompropionsäurealkylester der Formel III, verwendet. Als besonders vorteilhaft hat sich die Verwendung von 1 bis 3 Gew.% quaternäre Verbindung der Formel IV, bezogen auf den 2-Propionsäurealkylester der Formel III, erwiesen.

Geeignete quaternäre Verbindungen der Formel IV sind beispielsweise Tetrapropylammoniumchlorid, -bromid und -jodid, Tetrabutylammoniumchlorid, -bromid und -jodid, Benzyltrimethylammoniumchlorid, Benzyltriäthylammoniumchlorid, Benzyltributylammoniumchlorid, Benzyldimethylhexadecylammoniumchlorid, Butyltripropylammoniumbromid, Tributylmethylammoniumchlorid, Trioctylmethylammoniumchlorid, Phenyltrimethylammoniumchlorid oder -bromid, Phenyltriäthylammoniumchlorid, Tricaprylmethylammoniumchlorid, Decyltrimethylammoniumchlorid, Dodecyltrimethylammoniumchlorid, Hexadecyltrimethylammoniumchlorid, Hexadecylpyridiniumchlorid, Dodecylpyridiniumbromid, Octylpyridiniumbromid, Tetrabutylphosphoniumchlorid, -bromid und -jodid, Benzyltrimethylphosphoniumchlorid, Benzyltributylphosphoniumchlorid, Tributylmethylphosphoniumchlorid, Trioctylmethylphosphoniumbromid, Hexadecyltributylphosphoniumbromid, Decyltributylammoniumchlorid, Dodecyltriäthylammoniumbromid, Tetraphenylphosphoniumbromid, Hexadecyltrimethylphosphoniumbromid, Tributylmethylphosphoniumjodid und Tetraphenylphosphoniumbromid. Als besonders geeignet hat sich Tetrabutylammoniumbromid erwiesen.

Als säurebindende Mittel sind erfindungsgemäss Alkalicarbonate und Alkalihydrogencarbonate, wie Lithiumcarbonat, Natriumcarbonat, Kalliumcarbonat, Rubidiumcarbonat und Cäsiumcarbonat, sowie Lithiumhydrogencarbonat, Natriumhydrogencarbonat, Kaliumhydrogencarbonat, Rubidiumhydrogencarbonat und Cäsiumhydrogencarbonat geeignet. Bevorzugte säurebindente Mittel sind Natriumcarbonat, Kaliumcarbonat, Natriumhydrogencarbonat und Kaliumhydrogencarbonat. Die säurebindenden Mittel werden in der Regel in etwa stöchiometrischer Menge oder in einnem Ueberschuss eingesetzt. Vorteilhaft werden die säurebindenden Mittel in einer Menge von 1-3 Aequivalenten bezogen auf den zu bindenden Bromwasserstoff verwendet. Vorzugsweise verwendet man 1,1-1,3 Aequivalente säurebindentes Mittel bezogen auf den zu bindenden Bromwasserstoff.

Innerhalb des angegebenen Temperaturbereichs von 110-125 °C, in dem die Umsetzung eines 2,6-Dialkylanilins der Formel II mit einem 2-Brompropionsäurealkylester der Formel III durchgeführt wird, sind Temperaturen von 115 ° bis 120 °C bevorzugt. Das während der Umsetzung eines 2,6-Dialkylanilins der Formel II mit einem 2-Brompropionsäurealkylester der Formel III in Gegenwart eines Alkalicarbonats- bzw. -hydrogencarbonats gebildete Wasser wird vorteilhaft während der Umsetzung durch azeotrope Destillation abgetrennt.

Nach beendigter Umsetzung wird das Reaktionsgemisch abgekühlt und durch Extraktion mit Wasser von Salzen befreit. Anschliessend wird aus der organischen Phase das überschüssige 2,6-Dialkylanilin der Formel II durch Destillation abgetrennt. Die destillative Abtrennung des 2,6-Dialkylanilins der Formel II erfolgt vorteilhaft im Vakuum. Das als Rückstand erhaltene N-(1'-Alkoxycarbonyläthyl)-2,6-dialkylanilin der Formel I kann in der Regel als solches für weitere Umsetzungen, beispielsweise für die eingangs genannte Umsetzung mit Carbonsäurechloriden, verwendet werden. Falls ein spezieller Anwendungszweck dies erforderlich macht, kann das Produkt jedoch auch durch eine Vakuum-Rektification gereinigt werden.

Gemäss einer bevorzugten Ausführungsform des erfindungsgemässen Verfahrens setzt man pro Mol 2-Brompropionsäurealkylester der Formel III 1,7 Mol 2,6-Dialkylanilin der Formel II ein und führt die Umsetzung bei 115 ° bis 120 °C in Gegenwart von 1 bis 3 Gew.% Tetrabutylammoniumbromid bezogen auf die eingesetzte Menge 2-Brompropionsäurealkylester der Formel III und in Gegenwart von 0,55 bis 0,65 Mol Natriumcarbonat pro Mol 2-Brompropionsäurealkylester der Formel III durch. Nach dieser bevorzugten Ausführungsform lassen sich insbesondere 2,6-Dimethylanilin und 2-Brompropionsäuremethylester vorteilhaft umsetzen.

Mit dem erfindungsgemässen Verfahren wird es möglich, die N-(1'-Alkoxycarbonyl)-2,6-dialkylaniline der Formel I in höheren Ausbeuten und in besserer Qualität herzustellen als nach dem bisher bekannten Verfahren. Das erfindungsgemässe Verfahren kann auf einfache Weise in üblichen Apparaturen durchgeführt werden und eignet sich insbesondere für die kontinuierliche Durchführung. Im Hinblick auf die hohe Ausbeute und Qualität der Verfahrensprodukte und den geringen apparativen Aufwand kann das erfindungsgemässe Verfahren als besonders wirtschaftlich angesehen werden. Das erfindungsgemässe Verfahren bietet gegenüber bekannten Verfahren auch ökologische Vorteile, da infolge der hohen Ausbeute und der hohen Selektivität nur minimale Mengen an Zersetzungs- und Nebenprodukten ins Abwasser gelangen.

Das erfindungsgemässe Verfahren wird durch das folgende Beispiel näher erläutert.

Beispiel : Herstellung von N-(1'-Methoxycarbonyläthyl)-2,6-dimethylanilin

167,0 g (1 Mol) 2-Brompropionsäuremethylester, 206,0 g (1,7 Mol) 2,6-Dimethylanilin, 55,0 g (0,52 Mol) Natriumcarbonat und 5,0 g Tetrabutylammoniumbromid werden unter Rühren auf 115°-120 °C aufgeheizt und 6 Stunden bei dieser Temperatur gehalten. Dabei wird das gebildete Wasser während der Reaktion unter Vakuum abdestilliert. Nach gebildete Wasser während der Reaktion unter Vakuum abdestilliert. Nach beendigter Umsetzung wird das Reaktionsgemisch auf Raumtemperatur abgekült und mit 200 g Wasser verrührt bis alle Salze in Lösung sind. Nach dem Abtrennen der wässrigen Phase erhält man 202 g organische Phase, die zu etwa 33 Gew.% aus 2,6-Dimethylanilin und zu etwa 65 Gew.% aus N-(1'-Methoxycarbonyläthyl)-2,6-dimethylanilin besteht. Die destillative Trennung der organischen Phase ergibt 190,0 g (N-(1'-Methoxycarbonyläthyl)-2,6-dimethylanilin und 93,0 g 2,6-Dimethylanilin. Die Ausbeute an N-(1'-methoxycarbonyläthyl)-2,6-dimethylanilin beträgt 98,3 % bezogen auf umgesetztes 2,6-Dimethylanilin.

**Ansprüche**

1. Verfahren zur Herstellung von N-(1'-Alkoxycarbonyläthyl)-2,6-dialkylanilinen der Formel I

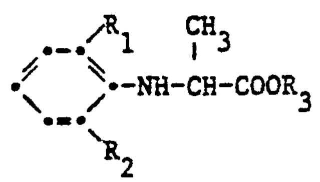

(I)

in welcher $R_1$ und $R_2$ je Methyl oder Aethyl bedeuten und $R_3$ für eine Alkylgruppe mit 1-4 Kohlenstoffatomen steht, durch Umsetzung eines 2,6-Dialkylanilins der Formel II

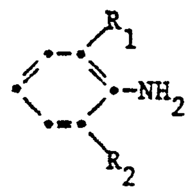

(II)

in welcher $R_1$ und $R_2$ die oben genannte Bedeutung haben, mit einem 2-Brompropionsäurealkylester der Formel III

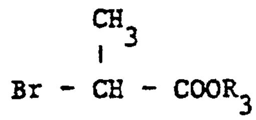

(III)

in welcher $R_3$ die oben angegebene Bedeutung hat, dadurch gekennzeichnet, dass man die Umsetzung des 2,6-Dialkylanilins der Formel II mit dem 2-Brompropionsäurealkylester der Formel III bei 110°-125 °C in überschüssigem 2,6-Dialkylanilinauflösungsmittel in Gegenwart einer quaternären Verbindung der Formel IV

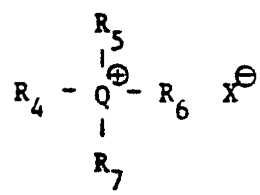

(IV)

in welcher Q Stickstoff oder Phosphor darstellt, die Reste $R_4$, $R_5$, $R_6$ und $R_7$ je einen Alkylrest mit 1 bis 16 Kohlenstoffatomen oder Phenyl bedeuten und einer der Reste $R_4$, $R_5$, $R_6$ und $R_7$ auch für Benzyl stehen kann, wobei, wenn Q Stickstoff bedeutet, Q zusammen mit dreien der Reste $R_4$, $R_5$, $R_6$ und $R_7$ auch einen Pyridinrest bedeuten kann, während der vierte Alkyl mit 1-16 Kohlenstoffatomen, Phenyl oder Benzyl darstellt, und $X^{\ominus}$ ein Halogenid- oder ein Bisulfatanion bedeutet und in Gegenwart eines Alkalimetallcarbonats oder -hydrogencarbonats als Säureakseptor führt, während der Umsetzung das gebildete Wasser abdestilliert und die nach Zugabe von Wasser und Phasentrennung erhaltene organische Phase destillativ aufarbeitet.

2. Verfahren nach Anspruch 1, dadurch gekennzeichnet, dass man das 2,6-Dialkylanilin der Formel II in einer Menge von 1,5 bis 2,5 Mol pro Mol 2-Brompropionsäurealkylester der Formel II einsetzt.

3. Verfahren nach Anspruch 1, dadurch gekennzeichnet, dass man das 2,6-Dialkylanilin der Formel II in einer Menge von 1,6 bis 1,8 Mol pro Mol 2-Brompropionsäurealkylester der Formel III einsetzt.

4. Verfahren nach Anspruch 1, dadurch gekennzeichnet, dass man die quaternäre Verbindung der Formel IV in einer Menge von 0,5 bis 5 Gew.% bezogen auf den 2-Brompropionsäurealkylester der Formel III einsetzt.

5. Verfahren nach Anspruch 1, dadurch gekennzeichnet, dass man die quaternäre Verbindung der Formel IV in einer Menge von 1 bis 3 Gew.% bezogen auf den 2-Brompropionsäurealkylester der Formel III einsetzt.

6. Verfahren nach Anspruch 1, dadurch gekennzeichnet, dass man als quaternäre Verbindung der Formel IV Tetrabutylammoniumbromid verwendet.

7. Verfahren nach Anspruch 1, dadurch gekennzeichnet, dass man als säurebindendes Mittel Natriumcarbonat, Kaliumcarbonat, Natriumhydrogencarbonat oder Kaliumhydrogencarbonat verwendet.

8. Verfahren nach Anspruch 1, dadurch gekennzeichnet, dass man die als säurebindende Mittel verwendeten Alkalicarbonate und -hydrogencarbonate in einer Menge von 1-3 Aequivalenten bezogen auf den zu bindenden Bromwasserstoff einsetzt.

9. Verfahren nach Anspruch 1, dadurch gekennzeichnet, dass man die als säurebindente Mittel zu verwendenden Alkalicarbonate und -hydrogencarbonate in einer Menge 1,1-1,3 Aequivalenten bezogen auf den zu bindenden Bromwasserstoff einsetzt.

10. Verfahren nach Anspruch 1, dadurch gekennzeichnet, dass man die Umsetzung eines 2,6-Dialkylanilins der Formel II mit einem 2-Brompropionsäurealkylester der Formel III bei einer Temperatur von 115° bis 120 °C durchführt.

11. Verfahren nach Anspruch 1, dadurch gekennzeichnet, dass man das bei der Umsetzung gebildete Wasser durch azeotrope Destillation abtrennt.

12. Verfahren nach Anspruch 1, dadurch gekennzeichnet, dass man pro Mol 2-Brompropionsäurealkylester der Formel III 1,7 Mol 2,6-Dialkylanilin der Formel II einsetzt und die Umsetzung bei 115° bis 120 °C in Gegenwart von 1-3 Gew.% Tetrabutylammoniumbromid bezogen auf den 2-Brompropionsäurealkylester der Formel III und in Gegenwart von 0,55 bis 0,65 Mol Natriumcarbonat pro Mol 2-Brompropionsäurealkylester der Formel III durchführt.

13. Verfahren nach Anspruch 1, dadurch gekennzeichnet, dass man als 2,6-Dialkylanilin der Formel II 2,6-Dimethylanilin verwendet.

14. Verfahren nach Anspruch 1, dadurch gekennzeichnet, dass man als 2-Brompropionsäurealkylester der Formel III 2-Brompropionsäuremethylester verwendet.

## Claims

1. A process for the preparation of N-(1'-alkoxycarbonylethyl)-2,6-dialkylanilines of the formula I

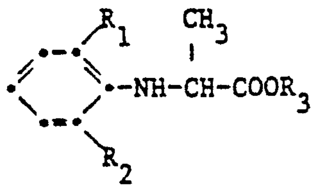

(I)

in which $R_1$ and $R_2$ are each methyl or ethyl and $R_3$ is an alkyl group having 1-4 carbon atoms, by reacting a 2,6-dialkylaniline of the formula II

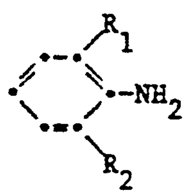

(II)

in wich $R_1$ and $R_2$ are as defined above, with an alkyl 2-bromopropionate of the formula III

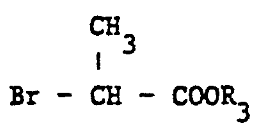

(III)

in wich $R_3$ is a defined above, which comprises carrying out the reaction of the 2,6-dialkylaniline of the formula II with the alkyl 2-bromopropionate of the formula III at 110°-125 °C, in excess 2,6-dialkylaniline as solvent, in the presence of a quaternary compound of the formula IV

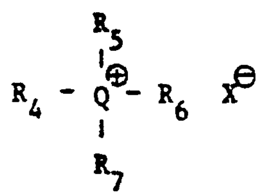

(IV)

in wich Q is nitrogen or phosphorus, the radicals $R_4$, $R_5$, $R_6$ and $R_7$ are each an alkyl radical having 1 to 16 carbon atoms, or phenyl, and one of the radicals $R_4$, $R_5$, $R_6$ and $R_7$ can also be benzyl, and, if Q is nitrogen, Q together with three of the radicals $R_4$, $R_5$, $R_6$ and $R_7$ can also be a pyridine radical, whilst the fourth radical is alkyl having 1-16 carbon atoms, phenyl or benzyl, and $X^{\ominus}$ is a halide anion or a bisulfate anion, and in the presence of an alkali metal carbonate or alkali metal bicarbonate as an acid acceptor, while distilling off the water formed during the reaction, and distilling the organic phase obtained after the addition of water and separation of the phases.

2. A process according to claim 1, wherein the 2,6-dialkylaniline of the formula II is employed in an amount of 1.5 to 2.5 moles per mole of alkyl 2-bromopropionate of the formula III.

3. A process according to claim 1, wherein the 2,6-dialkylaniline of the formula II is employed in an amount of 1.6 to 1.8 moles per mole of alkyl 2-bromopropionate of the formula III.

4. A process according to claim 1, wherein the quaternary compound of the formula IV is employed in an amount of 0.5 to 5 % by weight, based on the alkyl 2-bromopropionate of the formula III.

5. A process according to claim 1, wherein the quaternary compound of the formula IV is employed in an amount of 1 to 3 % by weight, based on the alkyl 1-bromopropionate of the formula III.

6. A process according to claim 1, wherein the quaternary compound of the formula IV employed is tetrabutylammonium bromide.

7. A process according to claim 1, wherein the acid acceptor used is sodium carbonate, potassium carbonate, sodium bicarbonate or potassium bicarbonate.

8. A process according to claim 1, wherein the alkali metal carbonate or alkali metal bicarbonate used as acid acceptor is employed in an amount of 1-3 equivalents, based on the hydrogen bromide to be bonded.

9. A process according to claim 1, wherein the alkali metal carbonate or alkali metal bicarbonate to be used as acid acceptor is employed in an amount of 1.1-1.3 equivalents, based on the hydrogen bromide to be bonded.

10. A process according to claim 1, wherein the reaction of a 2,6-dialkylaniline of the formula II with an alkyl 2-bromopropionate of the formula III is carried out at a temperature of 115 to 120 °C.

11. A process according to claim 1, wherein the water formed during the reaction is removed azeotropic distillation.

12. A process according to claim 1, wherein 1.7 moles of 2,6-dialkylaniline of the formula II are employed per mol of alkyl 2-bromopropionate of the formula III and the reaction is carried out at 115 ° to 120 °C in the presence of 1-3 % by weight of tetrabutylammonium bromide, based on the alkyl 2-bromopropionate of the formula III, and in the presence of 0.55 to 0.65 mole of sodium carbonate per mole of alkyl 2-bromopropionate of the formula III.

13. A process according to claim 1, wherein 2,6-dimethylaniline is used as 2,6-dialkylaniline of the formula II.

14. A process according to claim 1, wherein methyl 2-bromopropionate is used as alkyl 2-bromopropionate of the formula III.

**Revendications**

1. Procédé de préparation des N-(1'-alcoxycarbonyléthyl)-2,6-dialkylanilines de formule I

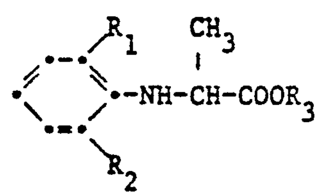

(I)

dans laquelle $R_1$ et $R_2$ représentent chacun un groupe méthyle ou éthyle et $R_3$ un groupe alkyle en C1-C4, par réaction d'une 2,6-dialkylaniline de formule II

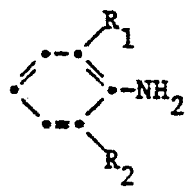

(II)

dans laquelle $R_1$ et $R_2$ ont les significations indiquées ci-dessus, avec un 2-bromo-propionate d'alkyle de formule III

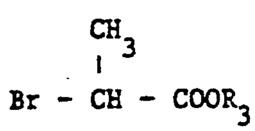

(III)

dans laquelle $R_3$ a la signification indiquée ci-dessus, caractérisé en ce que l'on effectue la réaction entre la 2,6-dialkylaniline de formule II et le 2-bromopropionate d'alkyle de formule III à des températures de 110 à 125 °C dans un excès de la 2,6-dialkylaniline qui sert de solvant, en présence d'un composé quaternaire de formule IV

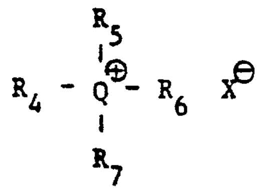

(IV)

dans laquelle Q représente l'azote ou le phosphore, les symboles $R_4$, $R_5$, $R_6$ et $R_7$ représentent chacun un groupe alkyle en C1-C16 ou phényle, l'un des symboles $R_4$, $R_5$, $R_6$ et $R_7$ pouvant également représenter un groupe benzyle, étant précisé que lorsque Q représente l'azote, Q peut également former avec trois des restes $R_4$, $R_5$, $R_6$ et $R_7$ un reste de pyridine, le quatrième représentant un groupe alkyle en C1-C16, phényle ou benzyle, et $X^{\ominus}$ représente un anion halogénure ou bisulfate, et en présence d'un carbonate ou bicarbonate de métal alcalin qui sert d'accepteur d'acide en distillant l'eau formée en cours de réaction, et en traitant par distillation la phase organique obtenue après addition d'eau et séparation des phases.

2. Procédé selon la revendication 1, caractérisé en ce que l'on utilise la 2,6-dialkylaniline de formule II en quantités de 1,5 à 2,5 moles par mole du 2-bromopropionate d'alkyle de formule III.

3. Procédé selon la renvendication 1, caractérisé en ce que l'on utilise la 2,6-dialkylaniline de formule II en quantités de 1,6 à 1,8 moles par mole du 2-bromopropionate d'alkyle de formule III.

4. Procédé selon la revendication 1, caractérisé en ce que l'on utilise le composé quaternaire de formule IV en quantités de 0,5 à 5 % du poids du 2-bromopropionate d'alkyle de formule III.

5. Procédé selon la revendication 1, caractérisé en ce que l'on utilise le composé quaternaire de formule IV en quantités de 1 à 3 % du poids du 2-bromopropionate d'alkyle de formule III.

6. Procédé selon la revendication 1, caractérisé en ce que l'on utilise en tant que composé quaternaire de formule IV le bromure de tétrabutylammonium.

7. Procédé selon la revendication 1, caractérisé en ce que l'on utilise en tant qu'agent fixant les acides le carbonate de sodium, le carbonate de potassium, le bicarbonate de sodium ou le bicarbonate de potassium.

8. Procédé selon la revendication 1, caractérisé en ce que les carbonates et bicarbonates alcalins utilisés en tant qu'agents fixant les acides sont mis en œuvre en quantités de 1 à 3 équivalents par rapport au bromure d'hydrogène à fixer.

9. Procédé selon la revendication 1, caractérisé en ce que les carbonates et bicarbonates alcalins à utiliser en tant qu'agents fixant les acides sont mis en œuvre en quantités de 1,1 à 1,3 équivalents par rapport au bromure d'hydrogène à fixer.

10. Procédé selon la revendication 1, caractérisé en ce que la réaction entre une 2,6-dialkylaniline de formule II et un 2-bromopropionate d'alkyle de formule III est effectuée à une température de 115 à 120 °C.

11. Procédé selon la revendication 1, caractérisé en ce que l'on sépare l'eau formée dans la réaction par distillation azéotropique.

12. Procédé selon la revendication 1, caractérisé en ce que, pour 1 mole du 2-bromopropionate d'alkyle de formule III, on utilise 1,7 moles de la 2,6-dialkylaniline de formule II et on effectue la réaction à une température de 115 à 120 °C en présence de 1 à 3 % en poids de bromure de tétrabutylammonium par rapport au 2-bromopropionate d'alkyle de formule III et en présence de 0,55 à 0,65 mole de carbonate de sodium par mole du 2-bromopropionate d'alkyle de formule III.

13. Procédé selon la revendication 1, caractérisé en ce que l'on utilise en tant que 2,6-dialkylaniline de formule II la 2,6-diméthylaniline.

14. Procédé selon la revendication 1, caractérisé en ce que l'on utilise en tant que 2-bromopropionate d'alkyle de formule III le 2-bromopropionate de méthyle.